# EUROPÄISCHE PATENTSCHRIFT

(11) **EP 0 932 393 B1**
(45) Veröffentlichungstag und Bekanntmachung des Hinweises auf die Patenterteilung: **03.04.2002**
(21) Anmeldenummer: 97944775.2
(22) Anmeldetag: 21.08.1997
(51) Int. Cl.: A61K 9/20

(54) **FESTE GESCHÄUMTE WIRKSTOFFZUBEREITUNGEN**
SOLID FOAMED ACTIVE SUBSTANCE PREPARATIONS
PREPARATIONS SOLIDES EXPANSEES DE PRINCIPES ACTIFS

(30) Priorität: 03.09.1996 DE 19635676
(43) Veröffentlichungstag der Anmeldung: 04.08.1999
(73) Patentinhaber: BASF AKTIENGESELLSCHAFT, 67056 Ludwigshafen (DE)
(72) Erfinder: BREITENBACH, Jörg, D-68199 Mannheim (DE); BAUMGARTL, Horst, D-55122 Mainz (DE)
(86) Internationale Anmeldenummer: EP9704550
(87) Internationale Veröffentlichungsnummer: WO9809616

(56) Entgegenhaltungen:
- EP-A- 0 240 904
- EP-A- 0 240 906
- EP-A- 0 694 376

## Beschreibung

Die vorliegende Erfindung betrifft feste, partiell oder vollständig geschäumte Wirkstoff-Formen auf Basis thermoplastisch verarbeitbarer Polymere.

Weiterhin betrifft die Erfindung Verfahren zur Herstellung solcher Wirkstoff-Formen.

Es ist allgemein bekannt, daß geschäumte Kunststoffe, durch Extrusion von flüchtigen Treibmitteln enthaltenden Schmelzen hergestellt werden können.

Weiterhin ist beispielsweise aus der EP-A 240 904 bekannt, feste Arzneiformen durch Extrusion wirkstoffhaltiger Polymerschmelzen mit anschließender Formgebung herzustellen.

Schnell freisetzende Arzneiformen werden in der Regel dadurch erzielt, daß durch die Verwendung von unlöslichen aber quellbaren Sprengmitteln eine schnelle Desintegration der Arzneiform herbeigeführt wird.

Eine schnelle Freisetzung kann bei Arzneiformen, die nach dem Verfahren der Schmelzextrusion erhalten werden, auch durch Einsatz relativ niedermolekularer, wasserlöslicher thermoplastischer Polymere als Matrixpolymere erzielt werden. Nachteilig daran ist aber, daß bei Verwendung solcher Polymere häufig Probleme bezüglich der Lagerstabilität der fertigen Arzneiformen aufweisen.

Aufgabe der vorliegenden Erfindung war es, wirkstoffhaltige Zubereitungen zu finden, die nach dem wirtschaftlich attraktiven Verfahren der Schmelzextrusion erhalten werden können und eine gezielte Freisetzung des Wirkstoffs ermöglichen.

Demgemäß wurden die eingangs definierten Wirkstoff-Formen gefunden. Weiterhin wurden Verfahren zur Herstellung solcher Wirkstoff-Formen gefunden.

Die erfindungsgemäßen festen geschäumten Wirkstoffzubereitungen können als Wirkstoffe alle Substanzen enthalten, die sich unter den Verarbeitungsbedingungen unzersetzt in die Polymerschmelze einarbeiten lassen.

Geeignete Wirkstoffe sind beispielsweise:

Acebutolol, Acetylcystein, Acetylsalicylsäure, Aciclovir, Alprazolam, Albumin, Alfacalcidol, Allantoin, Allopurinol, Ambroxol, Amikacin, Amiloride, Aminoessigsäure, Amiodarone, Amitriptyline, Amlodipine, Amoxicillin, Ampicillin, Ascorbinsäure, Aspartam, Astemizole, Atenolol, Azemetacin, Beclometason, Benscerazid, Benzalkonium Hydroxid, Benzocain, Benzoesäure, Betametason, Bezafibrate, Biotin, Biperiden, Bisoprolol, Bromazepam, Bromhexin, Bromocriptine, Budesonide, Bufexamac, Buflomedil, Buspiron, Coffein, Campher, Captopril, Carbamazepine, Carbidopa, Carboplatin, Cefachlor, Cefalexin, Cefadroxil, Cefazolin, Cefixime, Cefotaxime, Ceftazidine, Ceftriaxone, Cefuroxime, Chloramphenicol, Chlorhexidine, Chlorpheniramine, Chlortalidone, Choline, Ciclosporin, Cilastatin, Cimetidine, Ciprofloxacin, Cisapride, Cisplatin, Clarithromycin, Clavulanic Acid, Clomibramine, Clonazepam, Clonidine, Clotrimazole, Clozapin, Codeine, Colestyramine, Cromoglicinsäure, Cyanocobalamin, Cyproterone, Desogestrel, Dexamethasone, Dexpanthenol, Dexthromethorphan, Dextropropoxiphene, Diazepam, Diclofenac, Digoxin, Dihydrocodeine, Dihydroergotamine, Dilthiazem, Diphenhydramine, Dipyridamole, Dipyrone, Disopyramide, Domperidone, Dopamine, Doxocyclin, Enalapril, Enrofloxacin, Ephedrine, Epinephrine, Ergocalciferol, Ergotamine, Erythromycin, Estradiol, Ethinylestradiol, Etoposide, Eucalyptus Globulus, Famotidine, Felodipine, Fenofibrate, Fenoterol, Fentanyl, Flavin Mononucleotide, Fluconazole, Flunarizine, Fluorouracil, Fluoxetine, Flurbiprofen, Flutamid, Furosemide, Gemfibrozil, Gentamicin, Ginkgo Biloba, Glibenclamine, Glipizide, Glycyrrhiza Glabra, Guaifenesin, Haloperidol, Heparin, Hyaluronsäure, Hydrochlorothiazide, Hydrocodone, Hydrocortisone, Hydromorphon, Hydroxytetracyclin, Ipratropium Hydroxide, Ibuprofen, Imipenem, Indomethacin, Iohexol, Iopamidol, Isosorbide Dinitrate, Isosorbide Mononitrate, Isotredinoin, Kethotifen, Ketoconazole, Ketoprofen, Ketorolac, Labetalon, Lactulose, Lecithin, Levocarnitine, Levodopa, Levoglutamide, Levonorgestrel, Levothyroxine, Lidocaine, Lipase, Lisinopril, Loperamide, Lorazepam, Lovastatin, Medroxyprogesterone, Menthol, Methotrexate, Methyldopa, Methylprednisolone, Metoclopramide, Metoprolol, Miconazole, Midazolam, Minocycline, Minoxidil, Misobrostol, Morphine, Multivitamins and Minerals, Nystatin, N-Methylephedrine. Naftidrofuril, Naproxen, Neomycin, Nicardipine, Nicergoline, Nicotinamide, Nicotine, Nicotinic Acid, Nifedipine, Nimodipine, Nitrendipine, Nizatidine, Norethisterone, Norfloxacin, Norgestrel, Nortriphthyline, Ofloxacin, Omeprazole, Ondansetron, Pancreatin, Panthenol, Pantoprazol, Pantothenic Acid, Paracetamol, Penicillin G, Penicillin V, Phenobarbital, Phenoxifylline, Phenylephrine, Phenylpropanolamin, Phenytoim, Piroxicam, Polymyxin B, Povidone-Iod, Pravastatin, Prazepam, Prazosin, Prednisolone, Prednison, Proglumetacin, Propafenone, Propranolol, Pseudoephedrine, Pyridoxine, Quinidine, Ramipril, Ranitidine, Reserpine, Retinol, Riboflavin, Rifampicin, Rutoside, Saccharin, Salbutamol, Salcatonin, Salicyl Acid, Simvastatin, Somatropin, Sotalol, Spironolactone, Sucralfate, Sulbactam, Sulfamethoxazole, Sulpiride, Tamoxifen, Tegafur, Tenoxicam, Teprenone, Terazosin, Terbutaline, Terfenadine, Theophylline, Thiamine, Thiaprofensäure, Ticlopidine, Timolol, Tranexamsäure, Tretinoin, Triamcinolone Acetonide, Triamterene, Trimethoprim, Troxerutin, Uracil, Valproic Acid, Vancomycin, Verapamil, Vitamine E, Volinic Acid, Zidovudine, Zotepin.

Auch Vitamine lassen sich erfindungsgemäß formulieren. Dazu gehören die Vitamine der A-Gruppe, der B-Gruppe, wobei neben B1, B2, B6 und B12 sowie Nicotinsäure und Nicotinamid auch Verbindungen mit Vitamin B-Eigenschaften verstanden werden, wie z. B. Adenin, Cholin, Pantothensäure, Biotin, Adenylsäure, Folsäure, Orotsäure, Pangamsäure, Carnitin, p-Aminobenzoesäure, myo-Ionsit und α-Liponsäure. Weiterhin Vitamine der C-Gruppe, D-Gruppe, E-Gruppe, F-Gruppe, H-Gruppe,I- und J-Gruppe, K-Gruppe und P-Gruppe.

Ganz besonders bevorzugte Wirkstoffe sind erfindungsgemäß Ibuprofen, Acetylsalicylsäure, Paracetamol, Phenazon, Flurbiprofen, Captopril, Nifedipin, Acetylcystein, Naftidrofuryl, Verapamil und Furosemid.

Als Wirkstoffe kommen auch Pflanzenschutzmittel, andere Biocide oder veterinärmedizinische Substanzen in Betracht.

Als thermoplastisch verarbeitbare Polymere für die Polymermatrix kommen erfindungsgemäß amorphe, thermoplastische Polymere in Betracht.

Als Polymere eigenen sich vor allem wasserlösliche, thermoplastisch verarbeitbare Homo- oder Copolymere des N-Vinylpyrrolidons oder Gemische solcher Polymeren. Die Polymeren weisen üblicherweise Glasübergangstemperaturen im Bereich von 80 bis 190, bevorzugt 90 bis 175°C auf. Geeignete Homopolymere sind beispielsweise Polymere mit K-Werten nach Fikentscher im Bereich von 10 bis 30. Geeignete Copolymere können als Comonomere ungesättigte Carbonsäuren, z.B. Methacrylsäure, Crotonsäure, Maleinsäure, Itaconsäure, sowie deren Ester mit Alkoholen mit 1 bis 12, vorzugsweise 1 bis 8 Kohlenstoffatomen, ferner Hydroxyethyl- oder Hydroxypropylacrylat und -methacrylat, (Meth)acrylamid, die Anhydride und Halbester der Maleinsäure- und Itaconsäure (wobei der Halbester vorzugsweise erst nach der Polymerisation gebildet wird), oder Vinylmonomere wie N-Vinylcaprolactam, Vinylacetat, Vinylbutyrat und Vinylpropionat, enthalten oder auch Mischungen der genannten Comonomere. So eignen sich z.B. Terpolymere aus N-Vinylpyrrolidon, Vinylacetat und Vinylpropionat.

Bevorzugte Comonomere sind Acrylsäure und, besonders bevorzugt, Vinylacetat. Die Comonomere können in Mengen von 20 bis zu 70 Gew.-% enthalten sein. Ganz besonders bevorzugt sind erfindungsgemäß Copolymere, welche aus 60 Gew.-% N-Vinylpyrrolidon und 40 Gew.-% Vinylacetat erhalten werden.

Geeignete Polymere sind beispielsweise auch Homo- oder Copolymere des Vinylchlorids, Polyvinylalkohole, Polystyrol, Poly.....rate, Polyhydroxybutyrate oder Copolymere aus Ethylen und Vinylacetat.

Weiterhin können die Wirkstoffzubereitungen auch Stärken, abgebaute Stärken, Casein, Pektin, Chitin, Chitosan, Gelatine oder Schellack als Matrixkomponenten enthalten, welche unter Zusatz von üblichen Weichmachern in der Schmelze verarbeitet werden können.

Weiterhin können die erfindungsgemäßen Zubereitungen die üblichen pharmazeutischen Hilfsstoffe wie Füllstoffe, Schmiermittel, Formentrennmittel, Fließregulierungsmittel, Weichmacher, Farbstoffe und Stabilisatoren in Mengen bis zu ca. 50 Gew.-% enthalten. Diese und die im folgenden angegebenen Mengen sind jeweils bezogen auf das Gesamtgewicht der Zubereitung (= 100 %).

Als Füllstoffe seien z.B. die Oxide von Magnesium, Aluminium, Silicium und Titan sowie Lactose, Mannit, Sorbit, Xylit, Pentaerythrit und seine Derivate genannt, wobei die Menge an Füllstoff bei ca. 0,02 bis 50, vorzugsweise 0,2 bis 20 Gew.-% liegt.

Als Fließregulierungsmittel seien z.B. die Mono-, Di- und Triglyceride der langkettigen Fettsäuren wie C₁₂-, C₁₄-, C₁₆- und C₁₈-Fettsäure, Wachse wie Carnaubawachs sowie die Lecithine genannt, wobei die Menge bei ca. 0,1 bis 30, vorzugsweise 0,1 bis 5 Gew.-% liegt.

Als Weichmacher seien z.B. neben niedermolekularen Polyalkylenoxiden wie Polyethylenglykol, Polypropylenglykol und Polyethylenpropylenglykol auch mehrwertige Alkohole wie Propylenglykol, Glycerin, Pentaerythrit und Sorbit sowie Natriumdiethylsulfosuccinat, Mono-, Di- und Triacetat des Glycerin und Polyethylenglykolstearinsäureester genannt. Dabei liegt die Menge an Weichmacher bei ca. 0,5 bis 15, vorzugsweise 0,5 bis 5 Gew.-%.

Als Schmiermittel seien z.B. Stearate von Aluminium oder Calcium sowie Talkum und Silikone genannt, wobei ihre Menge bei ca. 0,1 bis 5, vorzugsweise 0,1 bis 3 Gew.-% liegt.

Als Stabilisatoren seien beispielsweise Lichtstabilisatoren, Antioxidantien, Radikalfänger und Stabilisatoren gegen mikrobiellen Befall genannt, wobei ihre Menge vorzugsweise bei ca. 0,01 bis 0,05 Gew.-% liegt.

Um die erfindungsgemäßen Zubereitungen herzustellen, kann die Wirkstoffkomponente entweder vorab mit dem Polymeren vermischt und anschließend extrudiert werden, oder aber im Laufe der Extrusion der treibmittelhaltigen Polymerschmelze zudosiert werden.

Die Mengenverhältnisse der einzelnen Komponente in der Zubereitung sind in weite Grenzen varierbar. Je nach Wirkdosis und Freisetzungsgeschwindigkeit des Wirkstoffs kann dessen Menge 0,1 bis 90 Gew.-% der Wirkstoffzubereitung betragen. Die Menge des Polymeren kann 10 bis 99,9 Gew.-% betragen. Zusätzlich können 0 bis 50 Gew.-% eines oder mehrerer Hilfstoffe enthalten sein.

Die Herstellung von vollständig geschäumten erfindungsgemäßen Wirkstoffzubereitungen erfolgt bevorzugt durch Extrusion einer Schmelze, welche neben einem oder mehreren Wirkstoffen eines oder mehrere thermoplastisch verarbeitbare Polymere sowie gegebenenfalls übliche Hilfstoffe enthält, wobei die Schmelze mit flüchtigen, physiologisch akzeptablen Treibmittel impragniert ist.

Als flüchtige, physiologisch akzeptable Treibmittel eignen sich gasförmige Treibmittel wie Kohlendioxid, Stickstoff, Luft, Edelgase wie beispielsweise Helium oder Argon, Chlorfluorkohlenwasserstoffe oder Distickstoffoxid (Lachgas), wobei Kohlendioxid und/oder Stickstoff bevorzugt werden.

Die Herstellung der Schmelze erfolgt vorzugsweise im Extruder, besonders bevorzugt in einem Zweischneckenextruder. Das Mischen des oder die Wirkstoffe mit den Polymeren und gegebenenfalls weiteren Zusätzen kann vor oder nach dem Schmelzen der Polymeren nach den in der Technik üblichen Verfahren erfolgen. Besonders bei temperaturempfindlichen Wirkstoffen empfiehlt es sich, diese erst nach dem Schmelzen des Thermoplasten zuzugeben. Die Schmelze kann bei Temperaturen von 50 bis 200, vorzugsweise 100 bis 180°C erhalten werden, wobei sich die geeignete Temperatur vor allem nach der Glasübergangstemperatur des oder der Polymere richtet.

Üblicherweise wird man die Polymeren bei Temperaturen oberhalb ihrer Glasübergangstemperatur aufschmelzen.

Die Imprägnierung der Schmelze mit dem Treibmittel erfolgt vorzugsweise bei Drücken von 10 bis 300 bar, besonders bevorzugt 50 bis 200 bar. Unter diesen Bedingungen lösen sich in der Schmelze zwischen 1 bis 15 Gew.-% des Treibmittels.

Durch die Imprägnierung mit weichmachenden Treibmitteln wie beispielsweise CO₂ erniedrigt sich die Viskosität der Schmelze, sodaß die Extrusion der treibmittelhaltigen Schmelze bei niedrigeren Temperaturen als bei einer entsprechenden treibmittelfreien Schmelze erfolgen kann. Diese Eigenschaft der treibmittelhaltigen Polymerschmelze begünstigt die Einarbeitung thermolabiler Wirkstoffe.

Vorzugsweise wird die treibmittelhaltige Schmelze auf Temperaturen abgekühlt, die im Bereich von 0 bis 50°C über der Glasübergangstemperatur des treibmittelfreien Polymeren oder Polymerengemischs liegt.

Bei besonders temperaturempfindlichen Wirkstoffen empfiehlt sich die Zugabe zur Schmelze nach der Treibmittelzumischung und erfolgter Temperaturabsenkung.

Das erfindungsgemäße Verfahren kann in einem einzigen Extruder mit unterschiedlichen Temperaturzonen durchgeführt werden. Bevorzugt wird jedoch eine Tandemextrusionsanlage bestehend aus zwei aneinandergekoppelten Extrudern, wobei der erste Extruder, in dem das Aufschmelzen des Polymeren und die Treibmittelbeladung der Schmelze erfolgt, vorzugsweise ein Zweiwellenextruder mit guter Mischwirkung ist, und der zweite Extruder ein Einwellenextruder mit geringer Scherwirkung und hoher Kühlleistung ist.

Der aus der Extruderdüse austretende noch plastische Strang expandiert unter dem außerhalb des Extruders vorliegende Normaldruck zu einem Schaum.

Der Verschäumungsgrad der Wirkstoffzubereitung kann über die Menge des zugesetzten Treibmittels und die Extrusionstemperatur gesteuert werden. Ein hoher Verschäumungsgrad hat eine niedrigere Dichte und damit eine hohe Auflösungsgeschwindigkeit der Wirkstofform zur Folge. Werden höhere Dichten gewünscht, so kann ein für die Zubereitung günstiger, hoher Treibmittelgehalt durch Entgasen unmittelbar zu dem Düsenspalt soweit abgesenkt werden, daß ein nur schwach aufgeschäumtes Produkt erhalten wird. Die geschäumte Wirkstoffzubereitung wird anschließend zu den jeweils gewünschten Wirkstoff-Formen verformt, beispielsweise durch Pelletierung, Granulierung oder Tablettierung nach bekannten Verfahren.

Die festen, vollständig geschäumten Wirkstoffzubereitungen weisen üblicherweise Dichten im Bereich von 200 bis 1000 g/l, vorzugsweise 200 bis 800 g/l auf.

Eine weitere Ausführungsform des erfindungsgemäßen Verfahrens betrifft die Herstellung von mehrschichtigen partiell oder vollständig geschäumten wirkstoffhaltigen Formen durch Koextrusion. Dabei werden mindestens zwei Massen, die jeweils mindestens eines der genannten thermoplastischen Bindemittel enthalten, von denen mindestens eine einen Wirkstoff enthält und von denen mindestens eine in der bereits geschilderten Weise mit einem gasförmigen physiologisch unbedeutlichen Treibmittel imprägniert ist, koextrudiert und anschließend zu oder gewünschten Darreichungsform verformt.

Vor der Koextrusion wird die Masse für jede Schicht der Wirkstofform separat zubereitet. Zu diesem Zweck werden die jeweiligen Ausgangskomponenten in einem separaten Extruder unter den bereits für die vorstehende Verfahrensvariante geschildertem Bedingungen zu wirkstoffhaltigen Schmelzen verarbeitet. Dabei kann für jede Schicht unter jeweils materialspezifisch optimalen Bedingungen gearbeitet werden. Beispielsweise kann für jede Schicht eine unterschiedliche Verarbeitungstemperatur gewählt werden. Die jeweiligen Massen können beispielsweise auch mit unterschiedlichen Mengen an Treibmittel imprägniert werden, sodaß Schichten mit unterschiedlichem Schäumungsgrad entstehen.

Die geschmolzenen oder plastischen Massen aus den einzelnen Extrudern werden in ein gemeinsames Koextrusionswerkzeug gefüllt, ausgeformt und ausgetragen. Die Form der Koextrusionswerkzeuge richtet sich nach der gewünschten Wirkstoff-Form. Beispielsweise sind Werkzeuge mit ebenem Austrittsspalt, sogenannte Breitschlitzwerkzeuge, und Werkzeuge mit kreisringspaltförmigen Austrittsquerschnitt geeignet. Die Düsenauslegung erfolgt dabei in Abhängigkeit von dem zur Anwendung kommenden polymeren Bindemittel und der gewünschten Form.

Nach dem Austrag aus dem Koextrusionswerkzeug erfolgt eine Formung zu der gewünschten Wirkstofform bzw. Arzneiform. Dabei kann eine Vielzahl von Formen je nach Koextrusionswerkzeug und Art der Formung erzeugt werden. Beispielsweise kann man aus einem Strang, der aus einem Breitschlitzwerkzeug austritt und der insbesondere zwei oder drei Schichten aufweist, durch Ausstanzen oder Ausschneiden z.B. mittels Glühdraht, offene Mehrschichttabletten herstellen. Alternativ können offene Mehrschichttabletten über ein Werkzeug mit kreisringspaltförmigem Austrittquerschnitt über einen Heißabschlag, d.h. durch zerschneiden bzw. Zerhacken des Stranges unmittelbar nach dem Austritt aus der Düse, oder vorzugsweise über einen Kaltabschlag, d.h. durch zerschneiden bzw. Zerhacken des Stranges nach zumindest teilweisem Abkühlen, separiert werden.

Geschlossene Wirkstoff-Formen, d.h. Formen, bei denen die wirkstoffhaltige Schicht vollständig von einer wirkstofffreien Schicht umgeben ist, erhält man insbesondere über ein Werkzeug mit kreisringspaltförmigem Austrittsquerschnitt durch Behandlung des Strages in einer geeigneten Quetschvorrichtung, wie sie beispielsweise in den Figuren 1 und 2, die in den nachfolgenden Beispielen erläutert wird, gezeigt ist. Dabei ist es von Vorteil, wenn bei bereits abgekühlter Außenschicht die Innenschicht der Mehrschichttablette beim Eintritt in die Quetschvorrichtung noch plastisch verformbar ist. Auf diese Weise lassen sich insbesondere Tabletten, vorzugsweise Oblong-Tabletten, Dragees, Pastillen und Pellets herstellen.

Nach einer weiteren Verfahrensvariante können geschäumte wirkstoffhaltige Formen in der Weise hergestellt werden, daß man eine Schmelze, die neben einem oder mehrerer Wirkstoffen mindestens ein thermoplastisches Bindemittel umfaßt, extrudiert, die noch plastische Schmelze einer Formgebung unterwirft und dann die feste wirkstoffhaltige Form unter Druck mit einem der oben genannten gasförmigen Treibmittel imprägniert, beispielsweise in einem herkömmlichen Autoklaven bei Drücken im Bereich von 10 bis 300 bar, vorzugsweise 50 bis 200 bar und anschließend expandiert. Bei Entspannen auf Normaldruck expandiert die imprägnierte Form zu einer partiell oder vollständig geschäumten Form.

Der Grad der Schäumung richtet sich nach der Dauer des Imprägniervorgangs und ist beliebig einstellbar. Diese Verfahrensvariante eignet sich bevorzugt zur Herstellung von partiell geschäumten Formen, die eine äußere geschäumte Hülle und einen nicht geschäumten Kern aufweisen und somit ein stufenförmiges Freisetzungsprofil aufweisen.

Die geschäumten Formen können auch mit einem üblichen wirkstoffdurchlässigen Überzug versehen werden, sodaß auf einfache Weise flotierende Schwimmformen erhalten werden können. Solche Schwimmformen können für pharmazeutische Zwecke oder auch für die veterinärmedizinische oder agrartechnische Produkte genutzt werden, beispielsweise für die Herstellung langsam sinkenden Fischfutters.

Die erfindungsgemäß erhaltenen, festen, geschäumten Wirkstoffzubereitungen, die den Wirkstoff homogen dispergiert in der polymeren Matrix enthalten, lösen sich sehr schnell auf und ermöglichen so die schnelle Freisetzung des Wirkstoffs. Durch das erfindungsgemäße Verfahren können die geschäumten Wirkstoffzubereitungen auf einfache und wirtschaftliche Art erhalten werden. Vorteilhaft ist auch, daß durch die viskositätsreduzierende Wirkung des Treibmittels bei deutlich niedrigeren Temperaturen als ohne Treibmittel extrudiert werden kann, sodaß die Wirkstoffe weniger thermisch belastet werden.

### Beispiel 1

Eine Mischung aus 20 Gew.-% Ibuprofen und 80 Gew.-% eines Polymeren aus 60 Gew.-% N-Vinyl-pyrrolidon und 40 Gew.-% Vinylacetat mit einem K-Wert von 30 wurde in einem Zweischneckenextruder bei 150°C aufgeschmolzen und bei einem Extruderinnendruck von 100 bar mit Kohlendioxid imprägniert, sodaß in der Schmelze 4 Gew.-% Kohlendioxid gelöst waren. In einem angeflanschten Einschneckenextruder wurde die treibmittelbeladene Schmelze auf 80°C abgekühlt und extrudiert. Durch den Austritt des Strangs in eine Normaldruckatmosphäre bei gleichzeitiger Abkühlung auf Raumtemperatur wurde eine feste geschäumte Wirkstoffzubereitung erhalten. Bei visueller Begutachtung unter einem Mikroskop zeigten die wirkstoffhaltigen Schäume in Wasser eine dreißigfach höhere Auflösungsgeschwindigkeit als eine entsprechende kompakte Wirkstofform, die durch Extrusion in Abwesenheit des Treibmittels erhalten wurde.

### Beispiel 2

Eine Mischung aus 26 Gew.-% Ibuprofen und 74 Gew.-% eines Copolymeren aus 60 Gew.-% N-Vinylpyrrolidon und 40 Gew.-% Vinylacetat mit einem K-Wert von 30 wurde in einem Zweischneckenextruder bei 150°C aufgeschmolzen und extrudiert. Der noch thermoplastische Strang wurde nach dem in der EPA 240 906 beschriebenen Verfahren zu Oblong-Tabletten mit einem Tablettengewicht von 650 mg verformt, die anschließend in einem Autoklaven bei 40°C und einem Kohlendioxiddruck von 100 bar vier Stunden lang imprägniert wurden. Anschließend wurde entspannt und auf Raumtempertur abgekühlt. Die Feisetzungsgeschwindigkeit wurde mit einer Paddle-Apparatur nach USP XII bestimmt. Nach 30 min waren 85 % des Wirkstoffs freigesetzt. im Vergleich dazu waren bei analog hergestellten Oblong-Tabletten, die nicht imprägniert und geschäumt wurden, nach 30 min nur 40 % des Wirkstoffs freigesetzt.

## Patentansprüche

1. Feste, partiell oder vollständig geschäumte Wirkstoff-Formen, enthaltend mindestens ein thermoplastisches Polymer.

2. Wirkstoff-Formen nach Anspruch 1, enthaltend als thermoplastisches Polymer ein Homo- oder Copolymer des N-Vinylpyrrolidon.

3. Wirkstoff-Formen nach Anspruch 1 oder 2, enthaltend einen Wirkstoff, der ausgewählt ist unter Ibuprofen, Ketoprofen, Flurbiprofen, Acetylsalicylsäure, Verapamil, Paracetamol, Nifedipin, Coffein, Captopril und Vitaminen oder Mischungen von zwei oder mehreren dieser Wirkstoffe.

4. Verfahren zur Herstellung von Wirkstoff-Formen gemäß einem der Ansprüche 1 bis 3, **dadurch gekennzeichnet, daß** man eine Schmelze, umfassend neben einem oder mehreren Wirkstoffen mindestens eine thermoplastisches Polymer und ein flüchtiges, physiologisch unbedenkliches Treibmittel, extrudiert und das expandierte Extrudat zu der gewünschten Wirkstofform verformt.

5. Verfahren zur Herstellung von Wirkstoff-Formen gemäß eine der Ansprüche 1 bis 3, **dadurch gekennzeichnet, daß** man mindestens zwei Schmelzen, die jeweils ein thermoplastisches Polymer umfassen von denen wenigstens eine einem oder mehrere Wirkstoffe enthält und von denen wenigstens eine ein physiologisch undenkliches Treibmittel enthält, koextrudiert und das expandierte Extrudat zu der gewünschten Wirkstofformen verformt.

6. Verfahren zur Herstellung von Wirkstoff-Formen gemäß einem der Ansprüche 1 bis 3, **dadurch gekennzeichnet, daß** man eine Schmelze, die neben einem oder mehreren Wirkstoffen mindestens ein thermoplastisches Bindemittel umfaßt, extrudiert, die noch plastische Schmelze einer Formgebung unterwirft, die feste Wirkstoff-Form unter Druck mit einem flüchtigen, physiologisch unbedenklichen Treibmittel imprägniert und anschließend expandiert.

7. Wirkstoff-Formen, erhältlich nach dem Verfahren gemäß Anspruch 4.

8. Wirkstoff-Formen, erhältlich nach dem Verfahren gemäß Anspruch 5.

9. Wirkstoff-Formen, erhältlich nach dem Verfahren gemäß Anspruch 6.

## Claims

1. A solid, partially or completely foamed active ingredient form comprising at least one thermoplastic polymer.

2. An active ingredient form as claimed in claim 1, comprising as thermoplastic polymer a homo- or copolymer of N-vinylpyrrolidone.

3. An active ingredient form as claimed in claim 1 or 2, comprising an active ingredient selected from ibuprofen, ketoprofen, flurbiprofen, acetylsalicylic acid, verapamil, paracetamol, nifedipine, caffeine, captopril and vitamins or mixtures of two or more of these active ingredients.

4. A process for producing active ingredient forms as claimed in any of claims 1 to 3, which comprises extruding a melt comprising, besides one or more active ingredients, at least one thermoplastic polymer and a volatile, physiologically acceptable blowing agent, and shaping the expanded extrudate to the required active ingredient form.

5. A process for producing active ingredient forms as claimed in any of claims 1 to 3, which comprises coextruding at least two melts, each of which comprises a thermoplastic polymer and at least one of which comprises one or more active ingredients and at least one of which comprises a physiologically acceptable blowing agent, and shaping the expanded extrudate to the required active ingredient forms.

6. A process for producing active ingredient forms as claimed in any of claims 1 to 3, which comprises extruding a melt which, besides one or more active ingredients, comprises at least one thermoplastic binder, subjecting the still plastic melt to a shaping, impregnating the solid active ingredient form under pressure with a volatile, physiologically acceptable blowing agent, and subsequently expanding.

7. An active ingredient form obtainable by the process as claimed in claim 4.

8. An active ingredient form obtainable by the process as claimed in claim 5.

9. An active ingredient form obtainable by the process as claimed in claim 6.

## Revendications

1. Formes solides, partiellement ou totalement gonflées en mousse, de substances actives, contenant au moins un polymère thermoplastique.

2. Formes de substances actives selon la revendication 1, contenant en tant que polymère thermoplastique un homo- ou co-polymère de la N-vinylpyrrolidone.

3. Formes de substances actives selon la revendication 1 ou 2, contenant une substance active choisie parmi l'Ibuprofen, le Ketoprofen, le Flurbiprofen, l'acide acétylsalicylique, le Verapamil, le Paracetamol, le Nifedipin, la caféine, le Captopril et des vitamines ou des mélanges de deux ou plusieurs de ces substances actives.

4. Procédé pour la préparation des formes de substances actives selon l'une des revendications 1 à 3, **caractérisé par le fait que** l'on extrude une masse fondue contenant, avec une ou plusieurs substances actives, au moins un polymère thermoplastique et un agent porogène volatil dépourvu de nocivité, et on met l'extrudat expansé sous la forme de substance active voulue.

5. Procédé de préparation des formes de substances actives selon l'une des revendications 1 à 3, **caractérisé par le fait que** l'on soumet à co-extrusion au moins deux masses fondues contenant chacune un polymère thermoplastique et dont l'une au moins contient une ou plusieurs substances actives et l'une au moins contient un agent porogène dépourvu de nocivité, et on met l'extrudat expansé sous la forme de substance active voulue.

6. Procédé pour la préparation des formes de substances actives selon l'une des revendications 1 à 3, **caractérisé par le fait que** l'on extrude une masse fondue contenant, avec une ou plusieurs substances actives, au moins un liant thermoplastique, on soumet la masse encore plastique à un formage, on imprègne la forme de substance active solide sous pression par un agent porogène volatil dépourvu de nocivité puis on soumet à expansion.

7. Formes de substances actives obtenues par le procédé selon la revendication 4.

8. Formes de substances actives obtenues par le procédé selon la revendication 5.

9. Formes de substances actives obtenues par le procédé selon la revendication 6.
